# EUROPÄISCHE PATENTSCHRIFT

(11) **EP 0 630 820 B1**
(45) Veröffentlichungstag und Bekanntmachung des Hinweises auf die Patenterteilung: **08.01.1997**
(21) Anmeldenummer: 94109105.0
(22) Anmeldetag: 14.06.1994
(51) Int. Cl.: B65B 55/02, A61L 2/26, A61B 19/00, G06K 7/10, G06F 17/00

(54) **Verfahren und System zur Überwachung des Materialflusses bei der Aufbereitung von Sterilgut**
Method and system for supervising the logistics when preparing sterile goods
Procédé et système pour la surveillance de la logistique de préparation des articles stérilisés

(30) Priorität: 14.06.1993 DE 4319403
(43) Veröffentlichungstag der Anmeldung: 28.12.1994
(73) Patentinhaber: C. STIEFENHOFER GmbH, D-86971 Peiting (DE)
(72) Erfinder: Kammermeier, Bernhard, D-82396 Fischen/Ammersee (DE); Filter, Michael, D-87616 Marktoberdorf (DE)
(74) Vertreter: Schaumburg, Thoenes & Thurn

(56) Entgegenhaltungen:
- DE-A- 3 917 876
- GB-A- 2 212 310

## Beschreibung

Die Erfindung betrifft ein Verfahren zum Überwachen des Materialflusses bei der Aufbereitung von Sterilgut gemäß dem Oberbegriff des Anspruchs 1 sowie ein System zur Durchführung eines solchen Verfahrens gemäß dem Oberbegriff des Anspruchs 12.

In Krankenhäusern wird Sterilgut verwendet. Sterilgut ist der Sammelbegriff für Hilfsmittel zur medizinischen Anwendung, die in einer Verpackung einem geeigneten auf Wirksamkeit kontrollierten Sterilisationsverfahren unterzogen werden. Eine Verpackungseinheit von Sterilgütern wird im folgenden Sterilisiereinheit genannt.

Unzureichende Prozeßbedingungen beim Sterilisiervorgang gefährden die hygienische Unbedenklichkeit der Sterilgüter und damit Leben und Gesundheit der damit behandelten Patienten.

Das Pflegepersonal im Krankenhaus bzw. der Krankenhausträger ist zu verantwortungsvollem Umgang mit dem fremden Rechtsgut "Leben und Gesundheit" des Patienten verpflichtet. Im Falle eines Regressanspruches durch einen geschädigten Patienten an das Krankenhaus oder das verantwortliche Personal tritt die sogenannte Beweislastumkehr in Kraft, d.h. der Beklagte muß unter anderem auch nachweisen, daß er hygienisch unbedenkliches Sterilgut für den Eingriff verwendet hat. Demzufolge besteht für den Krankenhausträger und das Krankenhauspersonal die Notwendigkeit, Hilfsmittel und Verfahrensweisen einzusetzen, die es ihnen ermöglichen, die einwandfreie hygienische Aufbereitung der Sterilgüter zweifelsfrei nachzuweisen.

Hinweise auf Daten, mit denen das Sterilgut gekennzeichnet werden soll, finden sich in der DIN 58953, Teil 9:
Sterilisierbehälter müssen so gekennzeichnet sein, daß deutlich zu erkennen ist, daß der Inhalt einem Sterilisationsverfahren unterworfen wurde. Zu diesem Zweck sind die Sterilisierbehälter mit einem Behandlungsindikator zu versehen. Es muß erkennbar sein, ob die Sterilisierbehälter vor der Verwendung des Sterilgutes geöffnet wurden. Darüber hinaus sind die Sterilisierbehälter
   a) mit dem Inhalt
   b) mit dem Sterilisationsdatum (Tag, Monat, Jahr)
   c) mit dem Namen und Signum des Verpackers zu kennzeichnen.
Alle Angaben müssen deutlich und auf einem Blick ersichtlich sein. Außerdem ist das Verfalldatum, das sich aus der Lagerdauer ergibt, kenntlich zu machen.

Nach dem derzeitigen Stand der Technik erfolgt die Kennzeichnung üblicherweise wie folgt:
Als Behandlungsindikator wird ein mit Indikatorfarbe bedrucktes Papierschild in die Packung eingelegt oder außen an dieser befestigt.
Zur Anzeige, ob die Packung geöffnet wurde, werden die Sterilisierbehälter plombiert oder versiegelt, wobei beim Öffnen des Behälters die Plomben oder Siegel zerstört werden.
Der Inhalt der Sterilisierbehälter wird durch außen an diesen befestigte Gravierschilder angezeigt. Bei Papier- oder Textilverpackungen wird häufig ein Klebestreifen angebracht und durch Filzschreiber beschriftet.
Das Sterilisationsdatum wird beispielweise von Hand auf ein Papierschild geschrieben, das an der Sterilisiereinheit außen befestigt wird. Dies ist in der Regel das gleiche Schild, das die Indikatorfarbe trägt. Das Datum kann auch mit einer Etikettierzange, wie sie in Warenhäusern zur Preisauszeichnung verwendet wird, auf ein Etikett gestempelt werden.
Das Signum des Verpackers wird als Unterschrift häufig auf dem Papierschild angebracht, das auch das Sterilisationsdatum trägt, oder es werden mehrfach verwendbare Blechmarken mit der Nummer des jeweiligen Verpackers in die Packung eingelegt.
Als Nachweis für die ordnungsgemäß durchgeführte Sterilisation werden die physikalischen Prozeßdaten der Sterilisation derzeit entweder mittels digitaler Aufzeichnungsgeräte (Meßdatendrucker) oder mittels analog arbeitender Aufzeichnungsgeräte aufgezeichnet. Dabei umfassen die aufgezeichneten Daten die Bezeichnung des gewählten Sterilisierprogrammes, das Sterilisationsdatum, eventuell eine Chargennummer und als Prozeßdaten die Verfahrenszeiten, - temperaturen und -drücke.

Bei den bislang modernsten Systemen werden nach der Sterilisation von einem Drucker am Sterilisator Packungsetiketten ausgegeben, welche die Daten "Sterilisationsdatum", "Identifikationsnummer des Sterilisators" und "Chargennummer" tragen. Diese Etiketten werden an den Sterilisiereinheiten angebracht. Die auf ihnen verzeichneten Daten können unter Umständen später ins Behandlungsprotokoll übertragen werden. Auch ein Verfalldatum kann auf dem Etikett vermerkt sein. Bei der Berechnung des Verfalldatums wird aber immer der gleiche Verfallzeitraum zugrundegelegt. Spezifische Daten wie Verpackungsart und Lagerbedingungen können nicht mit berücksichtigt werden, da der betreffenden Datenverarbeitungseinrichtung kein Bezug zum jeweiligen Sterilgut zur Verfügung steht. Diese Etiketten können auch nicht als individuelle Kennzeichnung der Sterilisiereinheiten angesehen werden, da die Sterilisiereinheiten einer Charge das gleiche Etikett tragen.

Beim Verbrauch gelangen die Sterilisiereinheiten zur Verwendungsstelle, die üblicherweise der Operationssaal ist. Unmittelbar vor der Verwendung wird das Packungsetikett nochmals kontrolliert. Zur Dokumentation der Behandlung des Patienten wird ein Behandlungsprotokoll erstellt. Die handgeschriebenen oder im Etikett enthaltenen Daten auf der Sterilisiereinheit werden handschriftlich auf das Behandlungsprotokoll übertragen. Das Behandlungsprotokoll wird für die gesamte Zeit, für die der Nachweis der hygienischen Unbedenklichkeit gefordert werden kann, aufbewahrt. In der Regel sind dies dreißig Jahre.

Falls nach der Behandlung ein Patient vom Krankenhaus oder dem verantwortlichen Personal den Nachweis der hygienischen Unbedenklichkeit der für seine Behandlung verwendeten Sterilgüter fordert, wird das Behandlungsprotokoll herangezogen und die Sterilisier-Chargennumer der verwendeten Sterilisiereinheit abgelesen. Aus den physikalischen Prozeßdaten, die auf dem dieser Nummer entsprechenden Chargendokument verzeichnet sind, soll die ordnungsgemäße Sterilisation und damit hygienische Unbedenklichkeit der Behandlungshilfsmittel aus der betreffenden Sterilisiereinheit nachgewiesen werden. Die vorstehend beschriebenen und sonstigen bisher bekannten Methoden der Dokumentation sind an vielen Stellen auf handschriftliche Aufzeichnungen angewiesen und dadurch arbeitsaufwendig und unsicher, da Kennzeichnungsfehler oder Manipulationen möglich sind. Dadurch wird jeder Nachweis der hygienischen Unbedenklichkeit der Sterilgüter anzweifelbar.

Ein wesentlicher Nachteil der bisher üblichen Methoden zur Gutkennzeichnung liegt darin, daß die Daten auf mehrere Datenträger verteilt sind und Verwechslungsfehler bei der Zuordnung geschehen können. Außerdem ist das Erstellen jedes Datenträgers mit zusätzlichem Arbeitsaufwand und zusätzlichen Kosten verbunden. Im folgenden werden eine Reihe möglicher Fehler aufgezählt:

Fehlermöglichkeiten am Verpackungsplatz:
Der Verpacker vergißt, den Indikator einzulegen (Fehler ist erst beim Verbrauch erkennbar).
Der Verpacker vergißt die Inhaltskennzeichnung.
Der Verpacker vergißt, das Sterilisierdatum auf das Papierschild zu schreiben, oder benutzt ein falsches Datum.
Der Verpacker vergißt, sein Signum anzubringen.

Fehlermöglichkeiten bei der Bedienung des Sterilisators:
Die Wahl des Sterilisationsprogrammes muß durch die Bedienungsperson des Sterilisators erfolgen. Dieser muß aufgrund der Beladung entscheiden, welches das richtige Programm ist. Die Bediener sind dabei häufig überfordert, insbesondere bei einer Mischbeladung des Sterilisators. Auch dem Personal im Operationssaal fehlt häufig die Beurteilungsfähigkeit, selbst wenn das Personal anhand eines eventuell vorhandenen Vermerks das angewandte Sterilisationsprogramm nochmal kontrollieren würde.
Falls nach der Sterilisation Etiketten vom Sterilisator ausgegeben werden, müssen diese auf den behandelten Sterilisiereinheiten angebracht werden. Dabei ergeben sich wieder Fehlermöglichkeiten. Vor der Ausgabe der Etiketten muß am Sterilisator die Zahl der auszudruckenden Etiketten eingegeben werden. Bei Fehleingabe können entweder einzelene Sterilisiereinheiten nicht etikettiert werden oder überzähliche Etiketten können mißbräuchlich verwendet werden. Ferner besteht die Möglichkeit, die Etiketten falsch zuzuordnen, d.h. der Charge X mit Etiketten der Charge Y zu bekleben.

Fehlermöglichkeit bei der Lagerung:
Da bei den bisher eingesetzten Dokumentationsmethoden eine Kennzeichnung des Lagerortes nicht üblich ist, besteht die Gefahr, daß die Güter unter Bedingungen gelagert werden, welche die vorausgeetzte Haltbarkeitsdauer nicht gewährleisten.

Fehlermöglichkeiten bei der erforderlichen Rückholaktion:
Falls der Verantwortliche der Sterilisationsabteilung nachträglich eine Funktionsstörung am Sterilisator erkennt und deshalb bestimmte nicht verwendbare Sterilisiereinheiten zurückholen muß, hat er keine zuverlässige Information über die Gutart, die Anzahl und den Verbleib der betreffenden Sterilisiereinheiten, es sei denn, daß zusätzliche handschriftliche Aufzeichnungen verfügbar sind, deren Erstellung jedoch wieder arbeitsaufwendig ist und die fehlerhaft sein können. Da bei den nicht zurückgeholten Gütern unter Umständen weder äußerlich noch am Indikator die Fehlfunktion des Sterilisationsprozesses erkannt werden kann, werden diese Einheiten möglicherweise verwendet und damit Patienten gefährdet.

Fehlermöglichkeiten bei der Übertragung der Daten:
Die Übertragung aller oder einzelner Daten von der Sterilisiereinheit auf das Behandlungsprotokoll erfolgt handschriftlich, wobei Übertragungsfehler möglich sind. Selbst für den Fall, daß bestimmte Daten durch ein vom Sterilisator ausgedrucktes und im Behandlungsprotokoll einklebbares Etikett übertragen werden, ist dennoch die korrekte Zuordnung möglicherweise zweifelhaft, da das gleiche Etikett auch von einer anderen Sterilisiereinheit mit einem ganz anderen Inhalt stammen könnte, das aber bei der gleichen Charge sterilisiert wurde.

Der Erfindung liegt die Aufgabe zugrunde, ein Verfahren der eingangs genannten Art anzugeben, das eine lückenlose Dokumentation der Aufbereitung von Sterilgut von der Verpackung bis zum Verbrauch ermöglicht, so daß der Nachweis der hygienischen Unbedenklichkeit des verwendeten Sterilgutes erbracht werden kann.

Diese Aufgabe wird erfindungsgemäß bei einem Verfahren der im Oberbegriff des Anspruchs 1 angegebenen Art dadurch gelöst, daß für die Sterilisiereinheit kennzeichnende Daten in eine mit dem Sterilisator verbundene Datenverarbeitungseinrichtung eingegeben werden, daß in Abhängigkeit von für die Sterilisiereinheit kennzeichnenden Daten ein geeignetes Sterilisationsverfahren ausgewählt wird und daß die für die Sterilisiereinheit und den Sterilisationsvorgang kennzeichnenden Daten gemeinsam gespeichert werden. Vorzugsweise werden die für die Sterilsiereinheit und den Sterilisationsvorgang kennzeichenden Daten auf einem Protokoll aufgezeichnet.

Bei dem erfindungsgemäßen Verfahren ist nun eine eindeutige Zuordnung eines bestimmten durch seine physikalischen Daten gekennzeichneten Sterilisationsverfahrens zu einer individuell gekennzeichneten Sterilisiereinheit möglich. Ferner ist sichergestellt, daß jede Sterilisiereinheit dem für das betreffende Sterilgut geeigneten Sterilisationsverfahren unterworfen wird.

Vorzugsweise sind dabei Daten auf dem mit den Sterilisiereinheiten verbindbaren Datenträgern und den Protokollen in maschinenlesbarer Form aufgezeichnet, so daß sie nicht von Hand übertragen werden müssen. Damit scheiden Fehler aus, die sich bei der Übertragung von Daten, auch der manuellen Eingabe von Daten in eine Dateneingabevorrichtung durch eine Bedienungsperson auftreten können.

Die individuelle Identifizierung der Sterilisiereinheiten schließt nicht aus, daß mehrere Sterilisiereinheiten vor dem Sterilisieren zu einer Charge zusammengefaßt werden, für die zusätzlich zu den den einzelnen Einheiten zugeordneten Datenträgern ein Sammeldatenträger mit den die Einheiten kennzeichnenden Daten erstellt wird. Für eine solche Charge brauchen nur die Daten des Sammeletiketts eingegeben oder eingelesen zu werden. Man vermeidet damit den Aufwand, der mit dem Eingeben oder Einlesen jedes einzelnen Etiketts verbunden ist.

Für eine Charge, die nicht notwendigerweise aus identischen Sterilisiereinheiten bestehen muß, wird ein Chargenprotokoll erstellt, auf dem sämtliche Sterilisiereinheiten identifizierbar aufgeführt sind, indem beispielsweise eine laufende Nummer für jede Sterilisiereinheit vergeben und aufgezeichnet wird.

Vorzugsweise sind die Datenträger, welche die für die jeweilige Sterilisiereinheit kennzeichnenden Daten tragen, lösbar mit den Sterilisiereinheiten verbindbar. Beispielsweise können die Datenträger als Klebeetiketten ausgebildet sein, so daß sie leicht an den Sterilisiereinheiten angebracht und auch von diesen wieder gelöst werden können, um sie beispielsweise auf den Behandlungsprotokollen aufzukleben. Damit wird die Notwendigkeit einer handschriftlichen Übertragung mit den dabei möglichen Fehlern vermieden.

Gegebenenfalls können auch Datenträger verwendet werden, die zwei Abschnitte umfassen, auf denen jeweils identische Datensätze aufgezeichnet sind. Einer der Abschnitte bleibt beim Verbraucher, wird also beispielsweise auf das Behandlungsprotokoll aufgeklebt, während der andere Abschnitt an der Sterilisiereinheit verbleibt. Damit kann auch der Rücklauf von wiederverwendbarem Sterilgut, also beispielsweise chirgurgischen Instrumenten überwacht werden.

Wenn die Datenträger für die Sterilisiereinheiten und die Protokolle mittels elektronischer Datenverarbeitungsgeräte erstellt werden, ist es vorteilhaft, wenn sich die Bedienungsperson an dem jeweiligen Gerät durch Eingabe einer Personenkennung ausweist, wobei die Personenkennung auf dem Datenträger bzw. dem Protokoll vermerkt wird. Dadurch können auch die für das Verpacken bzw. das Sterilisieren einer individuell benennbaren Sterilisiereinheit verantwortlichen Personen festgestellt werden.

Vorzugsweise werden bei der Abgabe einer sterilisierten Sterilisiereinheit an einen Verbraucher die Daten auf dem Datenträger nochmals erfaßt und zusammen mit den für den Verbraucher und die Umstände der Abgabe kennzeichnenden Daten gespeichert. Damit kann auch der Weg einer individuellen Sterilisiereinheit zum jeweiligen Verbraucher festgestellt werden. Darüber hinaus ist es möglich, diese Daten zur Lagerhaltung und Bestandsüberwachung zu verwenden. Schließlich kann bei dieser Gelegenheit das Verfallsdatum der jeweiligen Sterilisiereinheit überwacht und eine Ausgabe der Sterilisiereinheit verhindert werden, falls dieses Verfallsdatum bereits überschritten ist.

Das erfindungsgemäße Verfahren ermöglicht es also, jede Sterilisiereinheit individuell und unverwechselbar zu kennzeichnen, alle erforderlichen Daten zur patientenbezogenen Nachweisführung der sicheren Aufbereitung, Lagerung und bestimmungsgemäßen Verwendung von Sterilgütern zu dokumentieren, den Sterilisationsprozeß in Abhängigkeit der Beladung zu steuern und den Materialfluß der Sterilgüter zu kontrollieren.

Die Erfindung betrifft ferner ein System zur Überwachung des Materialflusses bei der Aufbereitung von Sterilgütern, umfassend eine Packstation zum Verpacken des zu sterilisierenden Gutes in einen Sterilgutbehälter, eine Sterilisationsstation mit einem Sterilisator zum Sterilisieren des Sterilgutes und ein Lager zum Aufbewahren und zur Ausgabe der jeweils aus dem Sterilgut und dem Sterilgutbehälter bestehenden Sterilisiereinheiten. Ein solches Systm ist erfindungsgemäß dadurch gekennzeichnet, daß die Packstation eine Dateneingabeeinrichtung zum Erfassen von für die Sterilguteinheiten kennzeichnenden Daten und eine Datenausgabeeinrichtung zum Aufzeichnen von für die Sterilisiereinheit kennzeichnenden Daten auf einem Datenträger hat, der an der Sterilisiereinheit befestigbar ist, daß der Sterilisator mit einer Datenverarbeitungseinrichtung verbunden ist, die eine Dateneingabeeinrichtung zum Erfassen mindestens eines Teiles der für die Sterilisiereinheit kennzeichnenden Daten und eine Datenausgabeeinrichtung hat, welche für die Sterilsiereinheit kennzeichnende Daten und den Sterilisationsvorgang betreffende Daten auf einem zweiten Datenträger aufzeichnet, und daß in dem Lager mindestens eine Dateneingabeeinrichtung zum Erfassen von auf dem ersten und/oder dem zweiten Datenträger aufgezeichneten Daten und eine Datenanzeigeeinrichtung zum Anzeigen von Kontrolldaten vorgesehen ist. An der Packstation und dem Lager können eigene Datenverarbeitungseinrichtungen vorgesehen sein. Vorzugsweise sind jedoch die Dateneingabe- und Datenausgabeeinrichtungen der Packstation und des Lagers mit der Datenverarbeitungseinrichtung an der Sterilisationsstation verbunden, so daß ein Datenaustausch unmittelbar erfolgen kann und die Daten nicht jedesmal beim Wechsel von einer Station zur anderen neu eingelesen werden müssen.

Vorzugsweise haben die Dateneingabeeinrichtungen Mittel zur Eingabe von Personenidentifizierungsdaten, so daß sich Bedienungspersonen an der Packstation und/oder der Sterilisationsstation und/oder dem Lager jeweils durch die Eingabe einer Personenkennung identifizieren können. Beispielsweise kann eine solche Einrichtung einen Kartenleser zur Eingabe einer Identifizierungskarte haben.

Vorzugsweise haben die Dateneingabeeinrichtungen jeweils eine Datenleseeinrichtung für maschinenlesbare Daten, beispielsweise eine Leseeinrichtung für Barcode oder einen Magnetstreifenleser, so daß Übertragungsfehler, die beim manuellen Eingeben von Daten auftreten können, vermieden werden.

Die Datenausgabeeinrichtung der Packstation ist vorzugsweise mit einem Etikettendrucker zum Bedrucken von Klebeetiketten ausgerüstet. Diese umfassen zweckmäßigerweise eine mit einer ersten Klebstoffschicht versehene Trägerschicht und eine Datenträgerschicht, die mittels einer zweiten Klebstoffschicht an der Trägerschicht lösbar haftet, wobei zweckmäßigerweise die Haftstärke der zweiten Klebstoffschicht auf der Trägerschicht geringer ist als die Haftstärke der ersten Klebstoffschicht auf einer Haftfläche des Sterilisiergutbehälters. Damit können die Klebeetiketten in der Packstation auf die Sterilgutbehälter geklebt werden. Beim Verbraucher werden dann die Datenträgerschichten abgezogen und beispielsweise auf ein Behandlungsprotokoll aufgeklebt.

Der erste Datenträger kann in an sich bekannter Weise einen auf einen Sterilisationsvorgang ansprechenden Behandlungsindikator tragen. Ferner kann der Datenträger derart mit dem Sterilgutbehälter verbunden sein, daß er erst nach dem Öffnen des Sterilgutbehälters von diesem lösbar ist.

Weitere Merkmale und Vorteile der Erfindung ergeben sich aus der folgenden Beschreibung, welche die Erfindung mit der beigefügten Zeichnung anhand eines Ausführungsbeispieles erläutert. Es zeigen:
- Figur 1: eine schematische Darstellung der Packstation und der in der Packstation erfolgenden Dokumentation,
- Figur 2: eine schematische Darstellung der Sterilisierstation und der in ihr erfolgenden Dokumentation,
- Figur 3: eine schematische Darstellung des Lagers für Sterilisiereinheiten und der mit ihrer Ausgabe verbundenen Vorgänge und
- Figur 4: eine schematische Darstellung der die Dokumentation des Sterilguts betreffenden Vorgänge in einem Operationssaal.

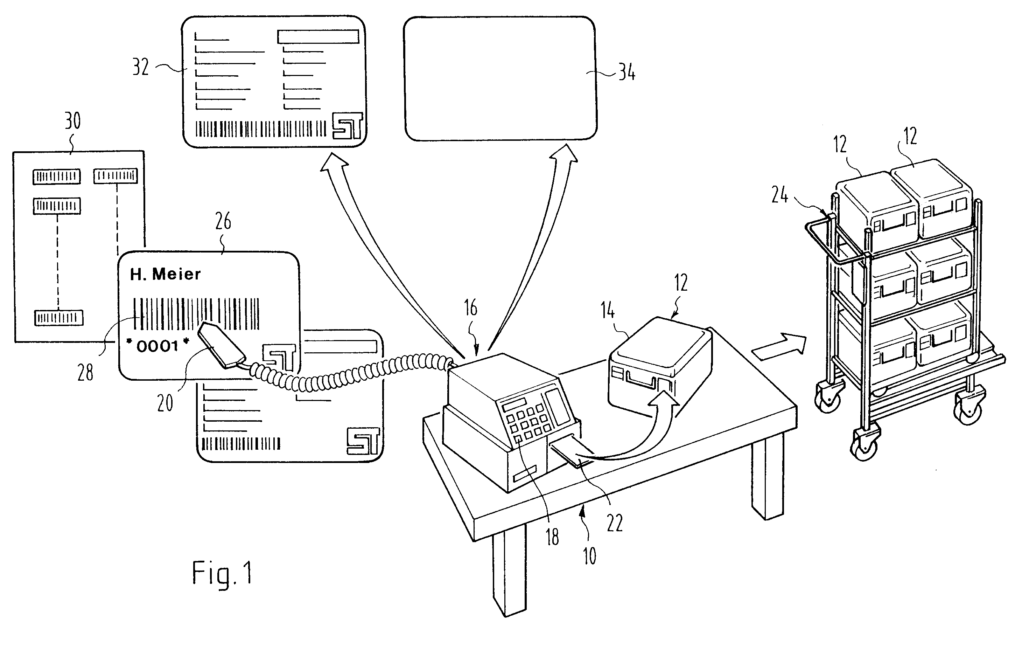

Figur 1 zeigt eine Verpackungsstation mit einem Packtisch 10, auf dem sich eine allgemein mit 12 bezeichnete Sterilisiereinheit befindet. Diese besteht aus einem Sterilgutbehälter 14 und dem darin enthaltenen Sterilgut. Auf dem Packtisch 10 befindet sich ferner ein Etikettendrucker 16 mit einer Eingabetastatur 18, einem Barcodelesestift 20 und einer Etikettenausgabe 22. Rechts von dem Verpackungstisch 10 befindet sich ein Transportwagen 24, auf dem eine Mehrzahl von Sterilisiereinheiten 12 zu einer Charge gesammelt werden.

Der Verpacker einer Sterilisiereinheit 12 nimmt üblicherweise auch die Etikettierung vor. Vor der Anforderung von Etiketten muß sich der Verpacker durch eine persönliche Codezahl identifizieren, die ihn zur Benutzung des Etikettendruckers 16 berechtigt. Hierzu hat er einen Ausweis 26, der seine als Barcode aufgedruckte persönliche Codezahl 28 trägt. Diese wird mit dem Lesestift 20 in den Etikettendrucker 16 eingelesen. Alternativ hierzu kann der Verpacker seine Codezahl auch über die Tastatur 18 am Etikettendrucker eingeben.

Der Verpacker wählt das gutartspezifische Etikett an, indem er am Etikettendrucker 16 die Artikelnummer des betreffenden Sterilgutes eingibt oder dieses von einem Artikelnummernverzeichnis 30 einliest, in dem die Artikelnummern in Form von Barcodes verzeichnet sind.

Der Etikettendrucker 16 gibt für jede Sterilisiereinheit 12 ein Packungsetikett 32 aus, das auf den Sterilgutbehälter 14 geklebt wird.

Am Etikettendrucker 16 kann auch eingegeben werden, daß eine ganze Reihe von einzelnen Packungsetiketten 32 zu einer Serie zusammengehören. Eine Serie ist üblicherweise die Summe aller Sterilisiereinheiten 12, die zu einer Chargenbeladung zusammengehören. Für diese Serie gibt der Etikettendrucker 16 nach der Ausgabe aller Packungsetiketten 32 automatisch ein Sammeletikett 34 aus, das der Serie zugeordnet wird und beispielsweise am Chargenwagen 24 angebracht wird. Ein Sammeletikett 34 repräsentiert immer nur eine Reihe von Packungsetiketten 32 einer einheitlichen Sterilgutart. Falls die Chargenbeladung aus einer Mischung von mehreren Gutarten besteht, werden dementsprechend viele Sammeletiketten 34 erzeugt, die aber dennoch zu einer Serie zusammengehören. Die zusammengehörigen Sammeletiketten 34 werden durchnumeriert und auf jedem Sammeletikett wird die Gesamtanzahl der zur Serie gehörenden Sammeletiketten ausgedruckt.

Der Etikettendrucker 16 enthält in seinem Datenspeicher die folgenden Daten:
Die Kennung (Codenummer) der bedienungsberechtigten Verpacker.
Sterilgutartspezifische Daten, die der jeweiligen Artikelnummer zugeordnet sind wie Artikelbezeichnung, zulässiger Verfallszeitraum, Name oder Funktion des Verbrauchers des jeweiligen Artikels und Nummer des vorgesehenen Lagerortes für den betreffenden Artikel.
Nichtsterilgutartspezifische Daten wie Identifikationsnummer des Etikettendruckers, Name der Verpackungsabteilung und Verpackungsdatum.
Errechnete Daten wie Verfallsdatum, das aus dem Verpackungsdatum plus dem sterilgutartspezifischen Verfallszeitraum ermittelt wird, laufende Nummer jedes Packungsetiketts 32, Start- und Endnummer der laufenden Nummern aller Packungsetiketten 32 einer zur Serie gehörenden Gutart und Nummer des jeweiligen Sammeletiketts 34 sowie Gesamtanzahl der Sammeletiketten 34 einer Serie.

Die laufende Nummer für jedes Packungsetikett 32 setzt sich dabei zusammen aus der Identifikationsnummer des Etikettendruckers und einer vom Etikettendrucker fortlaufend erhöhten Nummer. Die laufende Nummer muß immer die Identifikationsnummer des Etikettendruckers enthalten, da mehrere Drucker parallel arbeiten können und diese nicht im Datenverbund zueinander stehen müssen, so daß es bei der Vergabe einer laufenden Nummer, welche die Etikettendruckernummer nicht enthielte, zur Mehrfachvergabe derselben Nummer käme. Damit wäre eine individuelle Identifikation jeder Sterilisiereinheit nicht mehr gewährleistet.

Der Etikettendrucker 16 druckt auf die Packungsetiketten 32 eine Artikelnummer, die laufende Nummer der Sterilisiereinheit einschließlich der Identifikationsnummer des Etikettendruckers, das Verfalldatum und den Lagerort in einem Barcode verschlüsselt aus. Im Klartext aufgedruckt werden der Name der Verpackungsabteilung, der Name des Verpackers, der Name oder die Funktion des Verbrauchers, die Artikelbezeichnung und das Verpackungsdatum.

Auf die Sammeletiketten 34 druckt der Etikettendrucker die Artikelnummer, die Start- und Endnummer der laufenden Nummern aller Packungsetiketten 32 einer zur Serie gehörenden Sterilgutart, inclusive der Identifikationsnummer des Etikettendruckers, die Nummer des jeweiligen Sammeletiketts und die Gesamtanzahl der Sammeletiketten einer Serie, das Verfallsdatum und den Lagerort in Form eines Barcodes auf. Im Klartext werden ferner die Artikelbezeichnung und der Name des Verpackers aufgedruckt.

Die Packungsetiketten 32 können zusätzlich an einer nicht dargestellten geeigneten Stelle mit einer Indikatorfarbe bedruckt sein, die unter Dampfeinwirkung ihre Farbe ändert. Dadurch wird ersichtlich, ob eine Sterilisiereinheit bereits sterilisiert ist oder nicht.

Das Verpackungsetikett 32 verbleibt an der jeweiligen Sterilsiereinheit bis zu deren Benutzung. Die Etiketten 32 sind doppelt klebfähig und beständig gegen die Bedingungen, die während der Sterilisation auf sie einwirken.

Das Sammeletikett 34 ist ebenfalls dampfbeständig und verbleibt bei der zugehörigen Chargenladung bis zur unten zu beschreibenden Datenübernahme durch ein Strichcodelesegerät am Sterilisator. Es kann aber auch weiterhin als Datenträger benutzt werden bis zur Sterilgutausgabe oder auch bis zum Verbraucher, wenn die Chargenladung auf dem Weg dorthin nicht unterteilt wird.

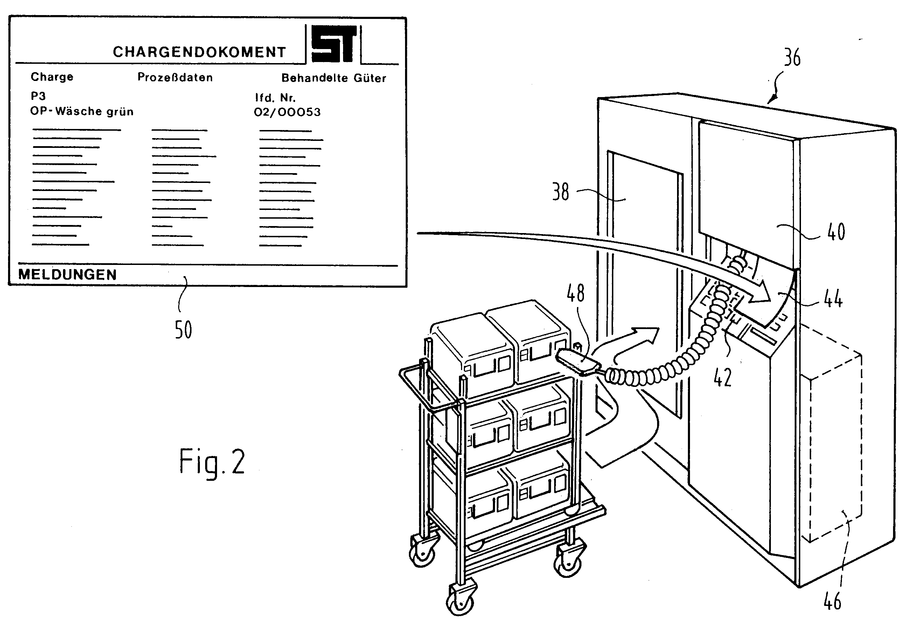

Figur 2 zeigt eine Sterilisationsstation mit einem Sterilisator 36. Dieser enthält eine durch eine Tür 38 verschließbare Sterilisationskammer, in welche der Transportwagen 24 mit den Sterilisiereinheiten 12 geschoben werden kann. Ferner umfaßt der Sterilisator 36 einen Steuerteil 40 mit einer Dateneingabeeinrichtung 42, einer Datenausgabeeinrichtung 44 und einer mit beiden in Verbindung stehende Datenverarbeitungseinrichtung 46, der im weiteren auch als Dokumentationsrechner bezeichnet werden soll. Die Dateneingabeeinrichtung 42 umfaßt eine nicht näher dargestellte Tastatur und einen Barcodelesestift 48. Der Dokumentationsrechner 46 könnte natürlich auch eine separate Einheit sein, die im Datenverbund mit dem Steuerteil 40 des Sterilisators 36 steht.

Der Dokumentationsrechner enthält in einem Datenspeicher mindestens die Kennung (Codenummern) der berechtigten Bedienungspersonen für den Sterilisator, die Zuordnung der Artikelnummern der verschiedenen Sterilgutarten zu den jeweiligen Sterilisierprogrammen, mit denen die Sterilgutarten ordnungsgemäß behandelt werden können, die Bezeichnung der Sterilisationsabteilung und die Identifikationsnummer des Sterilisators. Letztere ist wichtig, sofern mehrere Sterilisatoren parallel arbeiten.

In den Dokumentationsrechner 46 wird die Codenummer der jeweils tätigen Bedienungsperson eingegeben. Diese muß sich durch eine persönliche Codezahl identifizieren, welche sie zur Benutzung des Sterilisators 36 berechtigt. Dabei wird mit dem Barcodelesegerät 48 ähnlich wie an der Verpackungsstation ein Strichcodeausweis gelesen. Alternativ kann die Bedienungsperon ihre Codezahl auch über die Tastatur der Eingabeeinrichtung am Sterilisator 36 eingeben.

Ferner werden mit Hilfe des Barcodelesegerätes 48 die auf den Packungsetiketten 32 und/oder den Sammeletiketten 34 aufgedruckten Daten eingelesen. Aus den eingelesenen Daten der Packungsetiketten bzw. aus der Differenz zwischen Start- und Endnummer der laufenden Nummern auf den Sammeletiketten kann die Anzahl der Sterilisiereinheiten pro Gutart ermittelt werden. Da im Dokumentationsrechner 46 eine Zuordnung von bestimmten Sterilgutarten zu bestimmten Sterilisierprogrammen programmiert ist, kann der Dokumentationsrechner 46 automatisch das richtige Sterilisierprogramm auswählen und starten. Falls verschiedene Gutarten zu einer Charge zusammengestellt werden, für die kein gemeinsam taugliches Sterilisierprogramm verfügbar ist, wird eine entsprechende Fehlermeldung angezeigt. Während der Sterilisation fallen als Daten die Bezeichnung des gewählten Sterilisierprogrammes, das Sterilisationsdatum, die Chargennummer und Prozeßdaten wie Verfahrenszeiten, -temperaturen und -drücke an.

Der Dokumentationsrechner 46 ordnet die über das Barcodelesegerät 48 übernommenen oder auf andere Weise in den Dokumentationsrechner 46 eingegebenen Daten den im Speicher bereits vorhandenen und den während der Sterilisation anfallenden Daten zu. Am Ende des Sterilisationsprozesses werden diese Daten in Form eines Chargendokumentes 50 ausgegeben.

Auf dem Chargendokument 50 sind demnach neben den üblichen die Chargen kennzeichnenden Daten (Programmname, Chargennummer, Datum) und Prozeßdaten (Verfahrenszeiten, -temperaturen, -drücke) auch die für die spätere Rückverfolgung kennzeichnenden Daten aller beim betreffenden Prozeß behandelten Sterilisiereinheiten verzeichnet. Diese sind die Artikelnummern, die Stückzahlen pro Gutart (Artikel) sowie die laufende Nummer, das Verfalldatum und die Lageortnummer jeder Sterilisiereinheit.

Das Chargendokument 50 muß vom Verantwortlichen für die Sterilisation auf die ordnungsgemäßen Prozeßdaten hin kontrolliert und mit Unterschrift oder unverwechselbarem Signum abgezeichnet werden, bevor die Sterilisiereinheiten benutzt werden. Falls die Bedienungsperson Unregelmäßigkeiten erkennt, muß sie die Verwendung der betreffenden Sterilisiereinheiten sperren. Aufgrund der Daten auf dem Chargendokument 50 erkennt die Bedienungsperson die Anzahl, den Lagerort und die laufende Nummer jeder einzelnen Sterilisiereinheit und ist dadurch in der Lage diese wiederzufinden und zurückzuholen.

Das Chargendokument 50 wird für die gesamte Zeit, für die der Nachweis der hygienischen Unbedenklichkeit gefordert werden kann, aufbewahrt. Diese Zeit beträgt in der Regel dreißig Jahre.

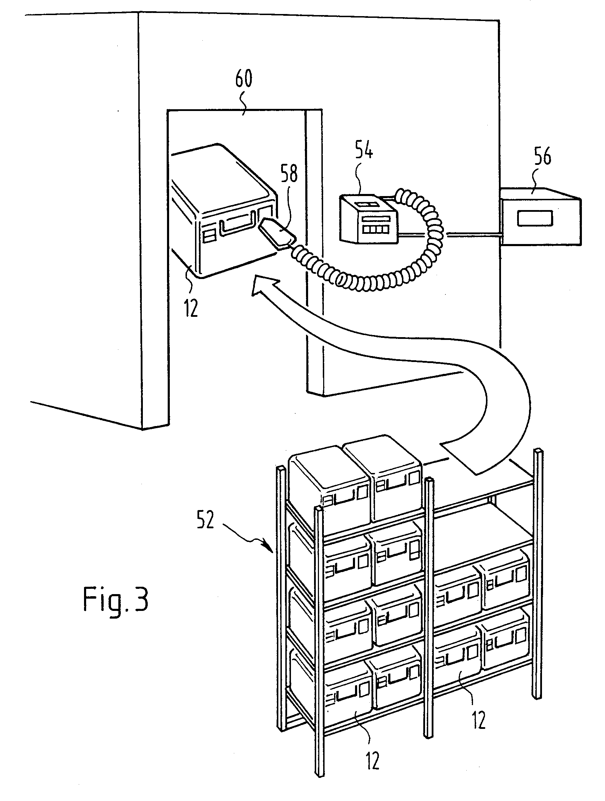

Nach erfolgreich abgeschlossener Sterilisation werden die Sterilisiereinheiten entladen und in die entsprechenden Lagerorte eines Sterilgutlagers 52 (Figur 3) eingeordnet. Im Dokumentationsrechner 46 wird dabei die Abgabe der entladenen Güter als Eingang in das Sterilgutlager 52 verbucht. Gegebenenfalls kann bei dieser Gelegenheit die Identität der Personen, welche die Entladung vornehmen, auf die gleiche Weise festgestellt werden, wie dies bei den verantwortlichen Personen an der Verpackungsstation und der Sterilisationsstation bereits beschrieben wurde.

Im Sterilgutlager 52 sind eine Dateneingabeeinrichtung 54 und eine Datenanzeigeeinrichtung 56 vorgesehen, die im Datenverbund mit dem Dokumentationsrechner 46 der Sterilisationsstation stehen. Die Dateneingabeeinrichtung 54 ist als Barcodelesegerät mit einem Lesestift 58 dargestellt. Selbstverständlich können auch andere Mittel zur Dateneingabe vorgesehen sein, beispielsweise ein Magnetstreifenleser oder dergleichen.

Vor dem Verlassen des Sterilgutlagers 52 wird das Packungsetikett 32 jeder Sterilisiereinheit mittels des Lesestiftes 58 gelesen. Falls nicht einzelne Sterilisiereinheiten 12 sondern eine komplette unzerteilte Charge ausgegeben wird, ist es vorteilhaft, an der Sterilgutausgabe 60 das bzw. die Sammeletiketten 34 zu lesen.

Der Dokumentationsrechner 46 kontrolliert anhand der eingelesenen Daten unmittelbar das Verfalldatum und vergleicht dieses mit dem abgespeicherten Verfallzeitraum. Falls es abgelaufen ist, wird an der Sterilgutausgabe 60 eine Fehlermeldung auf dem Datenanzeigegerät 56 angezeigt. Die betreffende Sterilisiereinheit darf dann nicht mehr ausgegeben werden.

Aufgrund der Tatsache, daß im Dokumentationsrechner 46 die kennzeichnenden Daten jeder Sterilisiereinheit 12 zum Zeitpunkt des Eingangs in das Sterilgutlager 52 und auch zum Zeitpunkt des Ausgangs erfaßt werden, ist es möglich, eine permanente Bilanzierung des Lagerbestandes durchzuführen. Der jeweils aktuelle Lagerbestand und die Bewegungen im Lager können am Dokumentationsrechner 46 ausgedruckt werden.

Falls mehrere Sterilisatoren 36 parallel arbeiten, ist es für die korrekte Lagerbestandsbilanzierung erforderlich, daß alle Sterilisatoren im Datenverbund mit einem Dokumentationsrechner stehen, der die von sämtlichen Sterilisatoren kommenden Sterilisiereinheiten bei ihrem Eingang ins Sterilgutlager 52 aufnimmt.

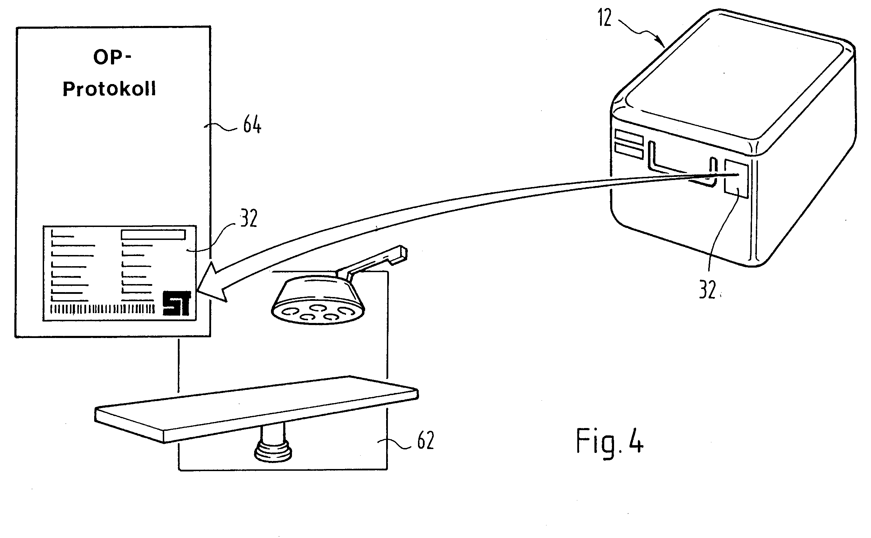

Der größte Teil des Sterilgutes wird in Operationssälen benötigt. In Figur 4 ist ein solcher durch einen Operationstisch 62 angedeutet. Bei oder nach Öffnung eines Sterilgutbehälters 14 wird das Packungsetikett 32 von dem Sterilgutbehälter 14 abgezogen und auf ein Operationsprotokoll 64 geklebt. Falls nach einer Behandlung oder einem operativen Eingriff ein Patient vom Krankenhaus oder dem verantwortlichen Personal den Nachweis der hygienischen Unbedenklichkeit der an ihm angewendeten Behandlungs- oder Operationsmittel fordert, wird das Behandlungsprotokoll 64 herangezogen. Die laufende Nummer des Packungsetiketts 32 der bei der Behandlung verwendeten Sterilisiereinheit 12 wird abgelesen.

Aus den abgelegten Chargendokumenten 50 des oder der Sterilisatoren 36 wird dasjenige herausgegriffen, auf dem die betreffende laufende Nummer verzeichnet ist. Durch die Bewertung der auf diesem Chargendokument 50 ebenfalls aufgezeichneten physikalischen Prozeßdaten kann die ordnunggemäße Sterilisation und damit die hygienische Unbedenklichkeit der Behandlungsmittel aus der betreffenden Sterilisiereinheit 12 nachgewiesen werden.

Damit ist die Rückverfolgbarkeit für jede Sterilisiereinheit von der Benutzung am Patienten zurück bis zur Aufbereitung geschlossen. Das Krankenhaus hat dadurch die Möglichkeit, sich vor ungerechtfertigten Schadensersatzansprüchen wegen Fehlern bei der Sterilgutaufbereitung zu schützen.

Bei dem Einsatz des erfindungsgemäßen Verfahrens und Systems sind zwar nach wie vor Fehler denkbar, jedoch ist die Zahl der möglichen Fehler erheblich vermindert und das Erkennen der noch möglichen Fehler erleichtert. So kann der Verpacker vergessen, ein Etikett auf die Sterilisiereinheit zu kleben. Dieser Fehler wird aber bereits beim Einlesen der Daten am Sterilisator 36 erkennbar. Dagegen kann der Verpacker nicht vergessen, den Behandlungsindikator, die Inhaltskennzeichnung, die Datumsangaben und sein Signum aufzubringen, da diese Kennzeichnungen entweder bereits im Etikett vorgegeben oder vor Ausgabe des Etiketts auf diesem aufgedruckt werden.

An der Sterilisationsstation könnte die Bedienungsperson vergessen, einzelne Packungsetiketten 32 einzulesen. Dieser Fehler kann jedoch nicht auftreten, falls Sammeletiketten verwendet werden, da auf jedem Sammeletikett verzeichnet ist, aus wievielen Sammeletiketten die Serie besteht. Der Sterilisator zeigt einen Fehler an, falls nicht alle Packungsetiketten eingelesen wurden.

Bei dem erfindungsgemäßen System sind Fehler wie die falsche Wahl des Sterilisierprogrammes, falsche Angaben über die Anzahl der Sterilsiereinheiten und die Zuordnung von Etiketten zu Gütern einer falschen Charge ausgeschlossen.

Aufgrund der Tatsache, daß auf jedem Packungsetikett 32 der korrekte Lagerort zu lesen ist, ist eine falsche Einlagerung sehr unwahrscheinlich.

Da jede Sterilisiereinheit individuell gekennzeichnet und auf dem Chargendokument 50 verzeichnet ist und da zudem der Verbleib (Lagerort oder Verbraucher) registriert ist, kann jede einzelne Sterilisiereinheit 12 ausfindig gemacht werden. Aufzeichnungslücken hinsichtlich der Anzahl der Sterilisiereinheiten sind praktisch nicht möglich. Dadurch ist bei Rückholaktionen sichergestellt, daß sämtliche zurückzuholenden Sterilisiereinheiten erfaßt werden können.

Die vorstehende Beschreibung zeigt, daß das erfindungsgemäße System eine praktisch lückenlose Dokumentation des Sterilgutes von der Aufbereitung bis zum Verbraucher sicherstellt. Es kann auch noch durch geeignete Maßnahmen sichergestellt werden, daß auch der Rücklauf von wiederverwendbarem Sterilgut wie beispielsweise Kleidung oder ärztliche Instrumente dokumentiert wird, so daß der Kreislauf des Sterilgutes geschlossen ist.

## Patentansprüche

1. Verfahren zum Überwachen des Materialflusses bei der Aufbereitung von Sterilgut, wobei Sterilgut in einem Sterilgutbehälter (14) verpackt wird, für die so gebildete Sterilisiereinheit (12) kennzeichnende Daten erfaßt und auf einem mit der Sterilisiereinheit (12) verbindbaren Datenträger (32) gespeichert werden, die Sterilisiereinheit (12) in einem Sterilisator (36) sterilisiert wird und die für den Sterilisiervorgang kennzeichnenden Daten erfaßt und aufgezeichnet werden, dadurch **gekennzeichnet**, daß für die Sterilisiereinheit (12) kennzeichnende Daten in eine mit dem Sterilisator (36) verbundene Datenverarbeitungseinrichtung (46) eingegeben werden, daß in Abhängigkeit von für die Sterilisiereinheit (12) kennzeichnenden Daten ein geeignetes Sterilisationsverfahren ausgewählt wird und daß die für die Sterilisiereinheit (12) und den Sterilisationsvorgang kennzeichnenden Daten gemeinsam gespeichert werden.

2. Verfahren nach Anspruch 1, dadurch **gekennzeichnet**, daß die für die Sterilisiereinheit (12) und den Sterilisationsvorgang kennzeichnenden Daten auf einem Protokoll (50) aufgezeichnet werden.

3. Verfahren nach Anspruch 1 oder 2, dadurch **gekennzeichnet**, daß die Daten auf den mit den Sterilisiereinheiten (12) verbindbaren Datenträgern (32, 34) und/oder den Protokollen (50) in maschinenlesbarer Form aufgezeichnet werden.

4. Verfahren nach einem der Ansprüche 1 bis 3, dadurch **gekennzeichnet**, daß mehrere Sterilisiereinheiten (12) vor dem Sterilisieren einer Sterilgutcharge zusammengefaßt werden, für die zusätzlich zu den den einzelnen Einheiten (12) zugeordneten Datenträgern (32) ein Sammeldatenträger (34) mit den die Einheiten (12) kennzeichnenden Daten erstellt wird.

5. Verfahren nach Anspruch 4, dadurch **gekennzeichnet**, daß für die mehrere Sterilguteinheiten (12) umfassende und demselben Sterilisationsvorgang unterworfene Sterilgutcharge ein Chargenprotokoll erstellt wird.

6. Verfahren nach einem der Ansprüche 1 bis 5, dadurch **gekennzeichnet**, daß die Datenträger (32) lösbar mit den Sterilguteinheiten (12) verbindbar sind.

7. Verfahren nach Anspruch 6, dadurch **gekennzeichnet**, daß als Datenträger (32) Klebeetiketten verwendet werden.

8. Verfahren nach Anspruch 6 oder 7, dadurch **gekennzeichnet**, daß der jeweilige Datenträger zwei Abschnitte umfaßt, auf denen identische Datensätze aufgezeichnet werden, wobei einer der Abschnitte beim Verbraucher des Sterilgutes von der Sterilisiereinheit entfernt wird, während der andere an der vom Verbraucher zur erneuten Sterilisierung zurückgegebenen Sterilisiereinheit verbleibt.

9. Verfahren nach einem der Ansprüche 1 bis 8, dadurch **gekennzeichnet**, daß die Datenträger (32) für die Sterilisiereinheiten (12) und die Protokolle (50) mittels elektronischer Datenverarbeitungsgeräte (16; 46, 44) erstellt werden, daß sich die Bedienungsperson an dem jeweiligen Gerät durch Eingabe einer Personenkennung ausweist und daß die Personenkennung auf dem Datenträger (32) bzw. dem Protokoll (34) aufgezeichnet wird.

10. Verfahren nach einem der Ansprüche 1 bis 9, dadurch **gekennzeichnet**, daß bei der Abgabe einer sterilisierten Sterilisiereinheit (12) an einen Verbraucher die Daten auf dem Datenträger (32) nochmals erfaßt und zusammen mit für den Verbraucher und die Umstände der Abgabe kennzeichnenden Daten gespeichert werden.

11. Verfahren nach Anspruch 10, dadurch **gekennzeichnet**, daß die bei der Abgabe der Sterilisiereinheit (12) an einen Verbraucher gespeicherten Daten an die Datenverarbeitungseinrichtung (46) des Sterilisators (36) übermittelt und dort anhand der für die jeweilige Sterilisiereinheit (12) bereits gespeicherten Daten geprüft werden und daß eine von dem Resultat der Überprüfung abhängige Freigabemeldung an die Abgabestelle übermittelt und eine Bestandskontrolle ausgeführt wird.

12. System zur Überwachung des Materialflusses bei der Aufbereitung von Sterilgut, umfassend eine Packstation zum Verpacken des zu sterilisierenden Gutes in einen Sterilgutbehälter (14), eine Sterilisationsstation mit einem Sterilisator (36) zum Sterilisieren des Sterilgutes und ein Lager (52) zum Aufbewahren und zur Ausgabe der aus dem Sterilgut und dem Sterilgutbehälter (14) bestehenden Sterilisiereinheit (12), dadurch **gekennzeichnet**, daß die Packstation eine Dateneingabeeinrichtung (10, 20) zum Erfassen von für die Sterilisiereinheit (12) kennzeichnenden Daten und eine Datenausgabeeinrichtung zum Aufzeichnen von für die Sterilisiereinheit kennzeichnenden Daten auf einem Datenträger (32) hat, der an der Sterilisiereinheit (12) befestigbar ist, daß der Sterilisator (36) mit einer Datenverarbeitungseinrichtung (46) verbunden ist, die eine Dateneingabeeinrichtung (42, 48) zum Erfassen mindestens eines Teils der für die Sterilisiereinheit (12) kennzeichnenden Daten und eine Datenausgabeeinrichtung (44) hat, die für die Sterilisiereinheit (12) kennzeichnende Daten und den Sterilisiervorgang betreffende Daten auf einen zweiten Datenträger (50) aufzeichnet, und daß in dem Lager (52) mindestens eine Dateneingabeeinrichtung (54, 58) zum Erfassen von auf dem ersten und/oder dem zweiten Datenträger aufgezeichneten Daten und eine Datenanzeigeeinrichtung (56) zum Anzeigen von Kontrolldaten vorgesehen ist.

13. System nach Anspruch 12, dadurch **gekennzeichnet**, daß die Dateneingabe- und ausgabeeinrichtung (16) der Packstation und/oder die Dateneingabe- und Datenanzeigeeinrichtung (54, 58; 56) des Lagers (52) mit der Datenverarbeitungseinrichtung (46) der Sterilisationsstation im Datenverbund stehen.

14. System nach Anspruch 12 oder 13, dadurch **gekennzeichnet**, daß die Dateneingabeeinrichtungen (18; 42; 54) Mittel zur Eingabe von Personenidentifizierungsdaten haben.

15. System nach einem der Ansprüche 12 bis 14, dadurch **gekennzeichnet**, daß die Dateneingabeeinrichtungen jeweils eine Datenleseeinrichtung (20; 48; 58) für maschinenlesbare Daten haben.

16. System nach einem der Ansprüche 12 bis 15, dadurch **gekennzeichnet**, daß die Datenausgabeeinrichtung (22) der Packstation ein Etikettendrucker ist.

17. System nach Anspruch 16, dadurch **gekennzeichnet**, daß die ersten Datenträger (32) Klebeetiketten sind, umfassend eine mit einer ersten Klebstoffschicht versehene Trägerschicht und eine Datenträgerschicht, die mittels einer zweiten Klebstoffschicht an der Trägerschicht lösbar haftet.

18. System nach Anspruch 17, dadurch **gekennzeichnet**, daß die Haftstärke der zweiten Klebstoffschicht auf der Trägerschicht geringer als die Haftstärke der ersten Klebstoffschicht auf einer Haftfläche des Sterilgutbehälters (14) ist.

## Claims

1. A method of monitoring the material flow in the processing of sterile material, the latter being packed in a sterile material container (14), data identifying the resulting sterilisation unit (12) being acquired and stored on a data support (32) adapted to be connected to the sterilisation unit (12), said unit (12) is sterilised in a steriliser (36) and the data identifying the sterilising operation are acquired and recorded, characterised in that data identifying the sterilisation unit (12) are input into a data processing device (46) connected to the steriliser (36), in that a suitable sterilisation process is selected according to the data identifying the sterilisation unit (12), and in that the data identifying the sterilisation unit (12) and the sterilisation operation are stored jointly.

2. A method according to claim 1, characterised in that the data identifying the sterilisation unit (12) and the sterilisation operation are recorded on a protocol (50).

3. A method according to claim 1 or 2, characterised in that the data are recorded in machine-readable form on the protocols (50) and/or the data supports (32, 34) connectable to the units (12) for sterilisation.

4. A method according to any one of claims 1 to 3, characterised in that before sterilisation of a sterile material charge a plurality of sterilisation units (12) are combined and a collective data support (34) in addition to the data supports (32) associated with the individual units (12) is prepared for the plurality of units and contains the data identifying said units (12).

5. A method according to claim 4, characterised in that a charge protocol is prepared for the sterile material charge comprising a plurality of sterile material units (12) and subjected to the same sterilisation operation.

6. A method according to any one of claims 1 to 5, characterised in that the data supports (32) are adapted to be releasably connected to the sterile material units (12).

7. A method according to claim 6, characterised in that adhesive labels are used as data supports (32).

8. A method according to claim 6 or 7, characterised in that each data support comprises two sections on which identical sets of data are recorded, one of the sections being removed by the sterile material consumer from the sterilisation unit, while the other remains on the sterilisation unit returned by the consumer for renewed sterilisation.

9. A method according to any one of claims 1 to 8, characterised in that the data supports (32) for the sterilisation units (12) and the protocols (50) are prepared by electronic data processing devices (16; 46, 44), in that the operator identifies himself at the associated device by inputting a personal identification, and in that the personal identification is recorded on the data support (32) or protocol (34).

10. A method according to any one of claims 1 to 9, characterised in that when a sterilised sterilisation unit (12) is delivered to a consumer the data on the data support (32) are again acquired and stored together with data identifying the consumer and the circumstances of delivery.

11. A method according to claim 10, characterised in that the data stored on delivery of the sterilisation unit (12) to a consumer is transmitted to the data processing device (46) of the steriliser (36), where it is tested against the data already stored for the associated sterilisation unit (12) and in that a release signal depending upon the result of the test is transmitted to the delivery station and an inventory control is carried out.

12. A system for monitoring the flow of material in the processing of sterile material, comprising a packing station for packing the material for sterilisation in a sterile material container (14), a sterilisation station with a steriliser (36) for sterilising the sterile material and a store (52) for storing and delivering the sterilisation unit (12) consisting of the sterile material and the sterile material container (14), characterised in that the packing station has a data inputting device (10, 20) for acquiring data identifying the sterilisation unit (12) and a data outputting device for recording on a data support (32) data identifying the sterilisation unit, said data support being adapted for fixing to the sterilisation unit (12), in that the steriliser (36) is connected to a data processing device (46) which has a data inputting device (42, 48) for acquiring at least some of the data identifying the sterilisation unit (12), and a data outputting device (44) which records on a second data support (50) data identifying the sterilisation unit (12) and data relating to the sterilisation operation, and in that the store (52) contains at least one data inputting device (54, 58) for acquiring data recorded on the first and/or the second data support, and a data display device (56) for displaying control data.

13. A system according to claim 12, characterised in that the data inputting and outputting device (16) of the packing station and/or the data inputting and data display device (54, 58; 56) of the store (52) are data-connected to the data processing device (46) of the sterilisation station.

14. A system according to claim 12 or 13, characterised in that the data inputting devices (18; 42; 54) have means for inputting personal identification data.

15. A system according to any one of claims 12 to 14, characterised in that the data inputting devices each have a data reader (20; 48; 58) for machine-readable data.

16. A system according to any one of claims 12 to 15, characterised in that the data outputting device (22) of the packing station is a label printer.

17. A system according to claim 16, characterised in that the first data supports (32) are adhesive labels, comprising a substrate provided with a first layer of adhesive, and a data support layer releasably adhering to the substrate by means of a second adhesive layer.

18. A system according to claim 17, characterised in that the adhesion of the second adhesive layer on the substrate is less than the adhesion of the first adhesive layer on an adhesive surface of the sterile material container (14).

## Revendications

1. Procédé pour la surveillance du flux de matériau pour la préparation d'articles à stériliser, les articles stérilisés étant conditionnés dans un conteneur pour articles stérilisés (14), les données caractéristiques pour l'unité de stérilisation (12) ainsi formée étant enregistrées et mémorisées sur un support de données (32) pouvant être relié à l'unité de stérilisation (12), l'unité de stérilisation (12) étant stérilisée dans un stérilisateur (36) et les données caractéristiques pour l'opération de stérilisation étant saisies et enregistrées, caractérisé en ce qu'on introduit des données caractéristiques pour l'unité de stérilisation (12) dans un appareil de traitement de données (46) relié au stérilisateur (36), en ce qu'on choisit un procédé de stérilisation approprié en fonction des données caractéristiques pour l'unité de stérilisation (12) et en ce que les données caractéristiques pour l'unité de stérilisation (12) et l'opération de stérilisation sont stockées en commun.

2. Procédé selon la revendication 1, caractérisé en ce que les données caractéristiques pour l'unité de stérilisation (12) et l'opération de stérilisation sont enregistrées sur un protocole (50).

3. Procédé selon la revendication 1 ou 2, caractérisé en ce que les données sont enregistrées sur les supports de données (32, 34) pouvant être reliés aux unités de stérilisation (12) et/ou les protocoles (50) dans une forme exploitable par une machine.

4. Procédé selon l'une quelconque des revendications 1 à 3, caractérisé en ce que plusieurs unités de stérilisation (12) sont regroupées avant la stérilisation d'une charge d'articles stérilisés, unités pour lesquelles on élabore un support de données collectives (34) avec les données caractérisant les unités (12) en supplément des supports de données (32) affectées aux différentes unités (12).

5. Procédé selon la revendication 4, caractérisé en ce qu'un protocole de charge est établi pour la charge d'articles stérilisés comprenant plusieurs unités d'articles stérilisés (12) et soumises à la même opération de stérilisation.

6. Procédé selon l'une quelconque des revendications 1 à 5, caractérisé en ce que les supports de données (32) peuvent être reliées de façon amovible aux unités d'articles stérilisés (12).

7. Procédé selon la revendication 6, caractérisé en ce que les étiquettes collantes sont utilisées comme support de données (32).

8. Procédé selon la revendication 6 ou 7, caractérisé en ce que le support de données concerné comprend deux parties sur lesquelles on enregistre des ensembles de données identiques, l'une des parties étant enlevée de l'unité de stérilisation chez l'utilisateur de l'article stérilisé, tandis que l'autre reste sur l'unité de stérilisation restituée par l'utilisateur pour la nouvelle stérilisation.

9. Procédé selon l'une quelconque des revendications 1 à 8, caractérisé en ce que le support de données (32) pour les unités de stérilisation (12) et les protocoles (50) sont établis au moyen d'appareils électroniques de traitement de données (16 ; 46, 44), en ce que l'opérateur décline son identité sur l'appareil concerné en introduisant un code d'identification de personne et en ce que le code d'identification de personne est enregistré sur le support de données (32) et le protocole (34).

10. Procédé selon l'une quelconque des revendications 1 à 9, caractérisé en ce que, lors de la remise d'une unité de stérilisation (12) stérilisée à un utilisateur, les données sont saisies une nouvelle fois sur le support de données (32) et stockées en même temps que les données caractéristiques pour l'utilisateur et les conditions de la remise.

11. Procédé selon la revendication 10, caractérisé en ce que les données stockées lors de la remise de l'unité de stérilisation (12) à un utilisateur sont transmises à l'appareil de traitement de données (46) du stérilisateur (36) et sont vérifiées ici à l'aide des données déjà stockées pour l'unité de stérilisation (12) concernée et en ce qu'un message d'autorisation dépendant du résultat du contrôle est transmis au centre de remise et un contrôle du stock est effectué.

12. Système pour la surveillance du flux de matériau lors de la préparation des articles stérilisés, comprenant une station d'emballage pour le conditionnement de l'article à stériliser dans un conteneur pour articles stérilisés (14), une station de stérilisation avec un stérilisateur (36) pour la stérilisation de l'article stérilisé et un entrepôt (52) pour la conservation et pour la sortie de l'unité de stérilisation (12) comprenant l'article stérilisé et le conteneur pour articles stérilisés (14), caractérisé en ce que la station d'emballage a un appareil d'entrée de données (10, 20) pour la saisie des données caractéristiques pour l'unité de stérilisation (12) et un appareil de sortie de données pour l'enregistrement de données caractéristiques pour l'unité de stérilisation sur un support de données (32), qui peut être fixé sur l'unité de stérilisation (12), en ce que le stérilisateur (36) est relié à un appareil de traitement de données (46) qui a un appareil d'entrée de données (42, 48) pour la saisie d'au moins une partie des données caractéristiques pour l'unité de stérilisation (12) et un appareil de sortie de données (44) qui enregistre des données caractéristiques pour l'unité de stérilisation (12) et des données concernant l'opération de stérilisation sur un deuxième support de données (50) et en ce que dans l'entrepôt (52) on prévoit au moins un appareil d'entrée de données (54, 58) pour la saisie de données enregistrées sur le premier et/ou le deuxième support de données et un appareil d'affichage de données (56) pour l'affichage de données de contrôle.

13. Système selon la revendication 12, caractérisé en ce que l'appareil d'entrée et de sortie de données (16) de la station d'emballage et/ou l'appareil d'entrée de données et l'appareil d'affichage de données (54, 58 ; 56) de l'entrepôt (52) se trouvent dans le circuit de données avec l'appareil de traitement de données (46) de la station de stérilisation.

14. Système selon la revendication 12 ou 13, caractérisé en ce que les appareils d'entrée de données (18 ; 42 ; 54) ont des moyens pour l'entrée de données d'identification de personne.

15. Système selon l'une quelconque des revendications 12 à 14, caractérisé en ce que les appareils d'entrée de données ont respectivement un appareil de lecture de données (20 ; 48 ; 58) pour des données exploitables à la machine.

16. Système selon l'une quelconque des revendications 12 à 15, caractérisé en ce que l'appareil de sortie de données (22) de la station d'emballage est une imprimante d'étiquettes.

17. Système selon la revendication 16, caractérisé en ce que les premiers supports de données (32) sont des étiquettes collantes, comprenant une couche support pourvue d'une première couche de colle et une couche de support de données qui adhère à la couche support au moyen d'une deuxième couche de colle.

18. Système selon la revendication 17, caractérisé en ce que la force d'adhérence de la deuxième couche de colle sur la couche support est plus faible que la force d'adhérence de la première couche de colle sur une surface d'adhérence du conteneur pour articles stérilisés (14).
